# EUROPÄISCHE PATENTANMELDUNG

(11) **EP 2 238 972 A2**
(43) Veröffentlichungstag der Anmeldung: **13.10.2010**
(21) Anmeldenummer: 10001298.8
(22) Anmeldetag: 09.02.2010
(51) Int. Cl.: A61K 9/16

(54) **Zusammensetzung umfassend einen hydrophoben Wirkstoff sowie ein Phospholipid**

(30) Priorität: 10.02.2009 DE 102009008185
(71) Anmelder: STADA ARZNEIMITTEL AG, 61118 Bad Vilbel (DE)
(72) Erfinder: Rillmann, Thomas, Dr., 76756 Bellheim (DE); Schönborn, Jessica, 61118 Bad Vilbel (DE); Schumann, Christof, 35767 Breitscheid-Erdbach (DE)
(74) Vertreter: Ackermann, Joachim

(57) **Zusammenfassung**

Die vorliegende Erfindung betrifft eine feste pharmazeutische Zusammensetzung, umfassend einen hydrophoben Wirkstoff, ein Phospholipid und ein Maltodextrin.

## Beschreibung

Die vorliegende Erfindung betrifft eine feste pharmazeutische Zusammensetzung, umfassend einen hydrophoben Wirkstoff, ein Phospholipid sowie ein Maltodextrin.

Phospholipide werden im Stand der Technik zur Verbesserung der Bioverfügbarkeit von Wirkstoffen vorgeschlagen. In vermarkteten pharmazeutischen Präparaten sind Phospholipide jedoch kaum anzutreffen, da entsprechende Zusammensetzungen den Nachteil einer verhältnismäßig geringen Stabilität aufweisen. So unterliegt insbesondere das Phospholipid selbst einer verhältnismäßig schnellen Zersetzung, was sich u.a. durch die Entwicklung eines unangenehmen Geruchs bemerkbar macht.

Es wäre wünschenswert, eine einen hydrophoben Wirkstoff ein Phospholipid umfassende feste pharmazeutische Zusammensetzung bereitstellen zu können, die eine verhältnismäßig hohe Stabilität aufweist und für die entsprechend eine längere Laufzeit festgelegt werden kann.

Aufgabe der vorliegenden Erfindung ist es daher, eine feste pharmazeutische Zusammensetzung bereitzustellen, die einen hydrophoben Wirkstoff sowie ein Phospholipid umfasst und die eine verhältnismäßig hohe Stabilität aufweist.

Diese Aufgabe wird gelöst durch eine feste pharmazeutische Zusammensetzung, umfassend einen hydrophoben Wirkstoff, ein Phospholipid sowie ein Maltodextrin.

Überraschenderweise wurde festgestellt, dass sich eine feste pharmazeutische Zusammensetzung, die einen hydrophoben Wirkstoff, ein Phospholipid sowie ein Maltodextrin umfasst, im Vergleich zu einer entsprechenden Zusammensetzung frei von Maltodextrin durch eine erhöhte Stabilität auszeichnet.

Unter dem Begriff "Stabilität" wird in diesem Zusammenhang die von der Arbeitsgemeinschaft für pharmazeutische Verfahrenstechnik (APV) erarbeitete Definition verstanden, nach welcher "Stabilität" die spezifikationsgerechte Qualität des Arzneimittels bis zum Ende der vom Hersteller festgelegten Laufzeit bedeutet. Die Qualität des Arzneimittels wird dabei durch den Wirkstoffgehalt und die Reinheit, die sensorisch wahrnehmbaren, physikalischchemischen und die mikrobiologischen Eigenschaften bestimmt, wobei der Wirkstoffgehalt bis zum Ende der Laufzeit 90 % des deklarierten Wertes nicht unterschreiten soll.

Die erfindungsgemäße Zusammensetzung zeichnet sich ferner durch eine verhältnismäßig gute Löslichkeit des Wirkstoffs in physiologischen Flüssigkeiten wie beispielsweise Wasser, Speichel oder gastrointestinalen Flüssigkeiten aus und stellt eine entsprechend hohe Bioverfügbarkeit des Wirkstoffs bereit.

Darüber hinaus ist die erfindungsgemäße Zusammensetzung auf verfahrenstechnisch einfache und damit kostengünstige Weise als Produkt mit einer einheitlichen gleichmäßigen Wirkstoffverteilung erhältlich, beispielsweise, wenn zur Herstellung der erfindungsgemäßen Zusammensetzung das Phospholipid in einer von Maltodextrin geträgerten, pulverigen Form eingesetzt wird. Von Maltodextrin geträgertes Phospholipid kann - in Abhängigkeit von dem Phospholipidanteil - als rieselfähiges trockenes Pulver vorliegen, das mit pulverförmigem hydrophobem Wirkstoff einfach zu einer Mischung verarbeitet werden kann, in welcher der Wirkstoff gleichmäßig verteilt enthalten ist. Phospholipide an sich hingegen sind in der Regel flüssig oder halbfest, z.B. gelartig. Ihr Einsatz in dieser Form ist verfahrenstechnisch problematisch und kostenintensiv.

Unter dem erfindungsgemäß verwendeten Ausdruck "hydrophober Wirkstoff" wird vorliegend eine pharmazeutisch wirksame Substanz verstanden, die bei einer Temperatur von 20 °C eine Löslichkeit in Wasser von kleiner als 1 g (Wirkstoff) pro 30 ml (Wasser) aufweist, oder ein pharmazeutisch annehmbares Salz davon. Das pharmazeutisch annehmbare Salz kann eine höhere Löslichkeit als die in der vorstehenden Definition angegebene aufweisen; entscheidend bei der Beurteilung, ob ein Wirkstoff unter die vorgenannte Definition des "hydrophoben Wirkstoffs" fällt, ist die Löslichkeit des freien Wirkstoffs in Wasser.

Alternativ dazu wird unter dem Ausdruck "hydrophober Wirkstoff" im Rahmen der vorliegenden Erfindung eine pharmazeutisch wirksame Substanz verstanden, die gemäß der Richtlinie "Guidance for Industry, Waiver of In Vivo Bioavailability and Bioequivalence Studies for Immediate-Release Solid Oral Dosage Forms Based on a Biopharmaceutics Classification System (BCS), U.S. Department of Health and Human Services, Food and Drug Administration (FDA), Center for Drug Evaluation and Research (CDER), August 2000, BP" unter Punkt II. als Wirksubstanz mit einer "Low Solubility" zu bezeichnen und entsprechend in die Klassen 2 oder 4 des BC-Systems einzuordnen ist, oder ein pharmazeutisch annehmbares Salz davon. Wirkstoffe der besagten Klasse 2 weisen neben einer geringen Löslichkeit in wässrigen Medien eine hohe intestinale Permeabilität auf, Wirkstoffe der Klasse 4 hingegen eine nur geringe. Die Methode zur Bestimmung der Löslichkeitsklasse eines Wirkstoffs ist an oben angegebenem Ort unter Punkt III. A unter der Überschrift "Determining Drug Substance Solubility Class" beschrieben.

Unter dem Ausdruck "pharmazeutisch annehmbares Salz" wird im Rahmen der vorliegenden Erfindung vorzugsweise ein therapeutisch wirksames, nichttoxisches Säureadditionssalz oder Basensalz der pharmazeutisch wirksamen Substanz verstanden.

Säureadditionssalze können durch Behandeln der Basenform einer pharmazeutisch wirksamen Substanz mit einer geeigneten anorganischen oder organischen Säure erhalten werden. Eine erfindungsgemäß bevorzugte anorganische Säure ist ausgewählt aus der Gruppe bestehend aus Halogenwasserstoffsäuren, insbesondere Salzsäure und Bromwasserstoffsäure, Schwefelsäure, Salpetersäure und Phosphorsäure. Eine bevorzugte organische Säure ist Essigsäure, Hydroxyessigsäure, Propansäure, Milchsäure, Brenztraubensäure, Oxalsäure, Malonsäure, Bernsteinsäure, Maleinsäure, Fumarsäure, Äpfelsäure, Weinsäure, Citronensäure, Mandelsäure, Methansulfonsäure, Ethansulfonsäure, Benzolsulfonsäure, p-Toluolsulfonsäure, Cyclamsäure, Salicylsäure, p-Aminosalicylsäure oder Pamoasäure.

Basensalze können durch Behandeln einer pharmazeutisch wirksamen Substanz, die acide Protonen enthält, mit einer geeigneten anorganischen oder organischen Base erhalten werden. Geeignete Basensalzformen der pharmazeutisch wirksamen Substanz sind vorzugsweise Ammoniumsalze, Alkali- und Erdalkalimetallsalze, insbesondere Lithium-, Natrium-, Kalium-, Magnesium- und Kalziumsalze, Benzathin-, N-Methyl-D-glucamin- und Aminosäuresalze, insbesondere Salze der Aminosäuren Arginin und Lysin.

Maltodextrin ist ein wasserlösliches Kohlenhydratgemisch, das durch Hydrolyse von Stärke erhältlich ist. Es enthält Monomere, Dimere, Oligomere und Polymere der Glukose. Je nach Hydrolysegrad unterscheidet sich der Anteil des Maltodextrins an Glukosemonomeren, -dimeren, -oligomeren und -polymeren, was durch das so genannte Dextrose-Äquivalent beschrieben werden kann, welches bei Maltodextrin in der Regel bei einem Wert von 3 bis 20 liegt. Maltodextrin ist die CAS.-Nr.: 9050-36-6 zugeordnet.

In der erfindungsgemäßen Zusammensetzung kann als Wirkstoff jeder hydrophobe pharmazeutische Wirkstoff eingesetzt sein.

Beispielsweise kann der hydrophobe Wirkstoff aus einer der nachfolgenden Wirkstoffklassen ausgewählt sein: Anästhetika, Analeptika, Analgetika, Anthelminika, Antiadiposita, Antiallergika, Antianämika, Antiarrhythmika, Antiasthmatika, Antibiotika, Antidementiva (Nootropica), Antidepressiva, Antidiabetika, Antidota, Antiepileptika, Antiemetika, Antihyperkinetika, Antihypertensiva, Antihypertonika, Antihypoglykämika, Antihypotonika, Antihypoxämika, Antimetastatika, Antimykotika, Antiphlogistika, Antirheumatika, Antitussiva, Antivertiginosa, Arteriosklerosemittel, β-Rezeptorenblocker, Broncholytika, Chemotherapeutika, Cholinergika, Diuretika, Emetika, Expectorantia, Fibrinolytika, Geriatrika, Gynäkologika, Hypnotika, Immuntherapeutika, Immunmodulatoren, Kalziumkanalblocker, Kardiaka, Lipidsenker, Lokalanästhetika, Muskelrelaxantia, Neuraltherapeutika, Neuroleptika, Ophthalmika, Opioid-Antagonisten, Opioid-Angonisten, Otologika, Parkinsonmittel, Psychopharmaka, Rhinologika, Seditiva, Sinusitismittel, Spasmolytika, Thrombocyten-Aggregationshemmer, Tuberkulosemittel, Urologika, Zystostatika.

Ein erfindungsgemäß bevorzugter hydrophober Wirkstoff ist vorzugsweise ausgewählt aus der Gruppe bestehend aus: Taxol, Codein, Diclofenac, Fentanyl, Hydromorphon, Ibuprofen, Indomethacin, Levomethadon, Morphin, Naproxen, Pritramid, Piroxicam, Anagrelid, Cabergolin, Clofibrat, Tramadol, Astemizol, Dimetinden, Doxylamin, Loratadin, Meclozin, Glimepirid, Pheniramin, Terfenadin, Erythromycin, Framycetin, Fusidinsäure, Rifampicin, Tetracyclin, Thiazetazon, Tyrothricin, Raloxifen, Carbamazepim, Clonazepam, Mesuximid, Phenytoin, Valproinsäure, Clotrimazol, Itraconazol, Darodipin, Isradipin, Aldosteron, Betametason, Budesonid, Glipizid, Dexametason, Fluocortolon, Paroxetin, Fludrocortison, Hydroxycortison, Methylprednisolon, Prednisolon, Cyclobarbital, Methaqualon, Phenobarbital, Azathioprin, Cyclosporin, Benzocain, Butanilacain, Etidocain, Lidocain, Oxybuprocain, Telmisartan, Tetracain, Modafinil, Dihydroergotamin, Ergotamin, Lisurid, Methysergid, Droperidol, Etomidat, Fentanyl, Ketamin, Methohexital, Propofol, Repaglinid, Thiopental, Acetazolamid, Betaxolol, Bupranolol, Carbachol, Carteolol, Cyclodrin, Cyclopentolat, Diclofenamid, Edoxodin, Homatropin, Levobununol, Pholedrin, Pindolol, Timolol, Tropicamid, Saquinavir, Indinavir, Ritonavir, Nelfinavir, Palinavir, Progesteron, Tamoxifen, Carmustin, Chlorambucil, Cyclophosphamid, Dacarbacin, Dactinomycin, Estramustin, Etoposid, Flurouracil, Ifosfamid, Methotrexat, Paclitaxel, Vinblastin, Vincristin, Vindesin und Ziprasidon. Insofern eine der vorstehenden Wirkstoffbezeichnungen ein Gemisch von Stereoisomeren einer pharmazeutisch wirksamen Substanz betrifft, so sind unter dem Ausdruck "hydrophober Wirkstoff" sowohl die einzelnen Stereoisomere als auch das Stereoisomeren-Gemisch zu verstehen.

Ein im Rahmen der vorliegenden Erfindung besonders bevorzugter hydrophober Wirkstoff ist ausgewählt aus der Gruppe bestehend aus Ziprasidon, Telmisartan und Raloxifen.

Der schwerlösliche Wirkstoff kann in der erfindungsgemäßen Zusammensetzung beispielsweise in kristalliner, semikristalliner oder amorpher Form enthalten sein sowie als Hydrat oder Anhydrat vorliegen. Dabei kann die Struktur der Wirkstoffpartikel regulär sein, irregulär, gebrochen oder sphärisch.

Entsprechend einer besonders bevorzugten Ausführungsform der erfindungsgemäßen Zusammensetzung ist der hydrophobe Wirkstoff Ziprasidon. Das Ziprasidon kann in Form der freien Base oder als pharmazeutisch annehmbares Salz davon in der Zusammensetzung enthalten sein.

Erfindungsgemäß bevorzugt liegt das Ziprasidon in Form des Hydrochlorid-Säureadditionssalzes in der Zusammensetzung vor. Dadurch wird eine bessere Löslichkeit des Wirkstoffes gewährleistet. Dabei liegt das Ziprasidonhydrochlorid vorzugsweise in Form des Monohydrates oder in Form des Anhydrates in der Zusammensetzung vor.

In der erfindungsgemäßen Zusammensetzung kann das Ziprasidon bzw. das pharmazeutisch annehmbare Salz davon grundsätzlich in jeder dem Fachmann für den erfindungsgemäßen Zweck geeigneten Form vorliegen. So ist es beispielsweise bevorzugt, dass der hydrophobe Wirkstoff in Form einer festen Lösung in der Zusammensetzung vorliegt, vorzugsweise in molekular-, nano- oder mikrodisperser Form. Dadurch wird eine verbesserte Löslichkeit des Wirkstoffs sichergestellt. Beispielsweise ist es erfindungsgemäß bevorzugt, dass das Ziprasidon bzw. das pharmazeutisch verträgliche Salz davon eine mittlere Partikelgröße von 1 nm bis 50 µm aufweist, vorzugsweise eine mittlere Partikelgröße von 0,1 µm bis 10 µm. Erfindungsgemäß besonders bevorzugt ist es aber, dass das Ziprasidon bzw. das pharmazeutisch annehmbare Salz davon eine mittlere Partikelgröße von größer als 85 µm aufweist, vorzugsweise eine mittlere Partikelgröße von größer als 85 µm bis zu 300 µm, bevorzugt eine mittlere Partikelgröße von 100 µm bis 180 µm, mehr bevorzugt eine mittlere Partikelgröße von 120 µm bis 150 µm und am meisten bevorzugt eine mittlere Partikelgröße von ungefähr 130 µm.

Unter "mittlere Partikelgröße" (D50-Wert) wird im Rahmen der vorliegenden Erfindung die Partikelgröße verstanden, bei der 50 % der Partikel bezogen auf das Volumen kleiner als der D50-Wert sind und 50 % der Partikel bezogen auf das Volumen größer sind als der D50-Wert. Die Bestimmung der mittleren Partikelgröße erfolgt mittels Laserbeugung und Einsatz eines Gerätes mit der Bezeichnung "Mastersizer 2000" der Firma "Malvem Instruments". Die Bestimmung des D50-Wertes wird erfindungsgemäß bevorzugt gemäß ISO/DIN 13320-1 durchgeführt, wobei die Messauswertung mittels der Fraunhofer-Methode erfolgt, die im Stand der Technik bekannt ist.

Liegt das Ziprasidon bzw. das pharmazeutisch annehmbare Salz davon in der erfindungsgemäßen Zusammensetzung in partikulärer Form vor, so ist der Wirkstoff vorzugsweise kristallin, semikristallin oder amorph.

Gemäß einer weiter bevorzugten Ausführungsform der erfindungsgemäßen Zusammensetzung ist es vorgesehen, dass die Zusammensetzung eine Säure umfasst. Es konnte festgestellt werden, dass in Gegenwart einer Säure die Löslichkeit des hydrophoben Wirkstoffs erhöht werden kann. Insbesondere Ziprasidon zeigt in Gegenwart einer Säure eine verbesserte Löslichkeit.

Entsprechend einer bevorzugten Ausführungsform der Erfindung beträgt das molare Verhältnis von der Säure zum Wirkstoff 0,1 bis 30, bevorzugt 1 bis 15, mehr bevorzugt 2 bis 10 und noch mehr bevorzugt 3 bis 5. Bei einem molaren Verhältnis von der Säure zum Wirkstoff unterhalb von 0,1 kann keine signifikante Erhöhung der Löslichkeit des Wirkstoffs beobachtet werden, oberhalb eines Molverhältnisses von 30 hingegen keine weitere Verbesserung der Löslichkeit.

Die in der erfindungsgemäßen Zusammensetzung enthaltene Säure kann im Prinzip jede Säure sein, die dem Fachmann für den erfindungsgemäßen Zweck als geeignet bekannt ist. Unter einer Säure wird dabei eine Verbindung verstanden, die im wässrigen Milieu zu einer Absenkung des pH-Wertes führt. Erfindungsgemäß bevorzugte Säuren sind Carbonsäuren, insbesondere Stearinsäure, Maleinsäure und Zitronensäure. Eine weitere erfindungsgemäß besonders bevorzugte Säure ist Ascorbinsäure oder Ameisensäure.

Weitere erfindungsgemäß bevorzugte Säuren sind Methacrylsäure-Methylmethacrylat-Copolymere, vorzugsweise mit einem Carboxyl- zu Estergruppen-Verhältnis von 1 bis 0,5. Derartige Produkte werden im Handel beispielsweise unter der Markenkennzeichnung Eudragit® L100 oder Eudragit® S100 angeboten. Eudragit® L100 weist ein Carboxyl- zu Estergruppen-Verhältnis von ungefähr 1 auf, Eudragit® S100 ein Carboxyl- zu Estergruppen-Verhältnis von ungefähr 0,5.

Entsprechend einer weiter bevorzugten Ausführungsform der erfindungsgemäßen Zusammensetzung ist der hydrophobe Wirkstoff Telmisartan. Telmisartan liegt vorzugsweise in freier Form oder als pharmazeutisch annehmbares Salz in der Zusammensetzung vor.

In der erfindungsgemäßen Zusammensetzung kann das Telmisartan bzw. das pharmazeutisch annehmbare Salz davon grundsätzlich in jeder dem Fachmann für den erfindungsgemäßen Zweck geeigneten Form vorliegen. So ist es beispielsweise bevorzugt, dass der hydrophobe Wirkstoff in Form einer festen Lösung in der Zusammensetzung vorliegt, vorzugsweise in molekular-, nano- oder mikrodisperser Form. Dadurch wird eine verbesserte Löslichkeit des Wirkstoffs sichergestellt. Beispielsweise ist es erfindungsgemäß bevorzugt, dass Telmisartan bzw. das pharmazeutisch verträgliche Salz davon eine mittlere Partikelgröße von 1 nm bis 50 µm aufweist, vorzugsweise eine mittlere Partikelgröße von 0,1 µm bis 10 µm.

Gemäß einer weiter bevorzugten Ausführungsform der erfindungsgemäßen Zusammensetzung ist es vorgesehen, dass die Zusammensetzung eine Base umfasst. Es konnte gezeigt werden, dass in Gegenwart einer Base die Löslichkeit des hydrophoben Wirkstoffs verbessert werden kann. Insbesondere Telmisartan zeigt in Gegenwart einer Base eine verbesserte Löslichkeit.

Entsprechend einer weiter bevorzugten Ausführungsform der Erfindung beträgt das molare Verhältnis von der Base zum Wirkstoff 0,1 bis 30, bevorzugt 1 bis 15, mehr bevorzugt 2 bis 10 und noch mehr bevorzugt 3 bis 5. Bei einem molaren Verhältnis von der Base zum Wirkstoff unterhalb von 0,1 kann keine signifikante Erhöhung der Löslichkeit des Wirkstoffs beobachtet werden, oberhalb eines Molverhältnisses von 30 hingegen keine weitere Verbesserung der Löslichkeit.

Die in der erfindungsgemäßen Zusammensetzung enthaltene Base kann im Prinzip jede Base sein, die dem Fachmann für den erfindungsgemäßen Zweck als geeignet bekannt ist. Unter einer Base wird dabei eine Verbindung verstanden, die im wässrigen Milieu zu einer Erhöhung des pH-Wertes führt.

Eine erfindungsgemäß bevorzugte Base ist eine Base ausgewählt aus der Gruppe bestehend aus Arginin, Lysin, Histidin und Meglumin. Darüber hinaus ist es erfindungsgemäß auch bevorzugt, dass in der Zusammensetzung ein Gemisch von zwei oder mehr voneinander verschiedener Basen enthalten ist, insbesondere ein Gemisch von zwei oder mehr der vorgenannten Basen.

Weitere erfindungsgemäß bevorzugte Basen sind basisch reagierende Copolymere enthaltend Aminomethacrylat- und/oder Aminoalkylmethacrylat-Einheiten. In diesem Zusammenhang erfindungsgemäß besonders bevorzugte Copolymere sind Copolymere enthalten Dimethylaminoethylmethacrylat-, Methylmethacrylat- und Butylmethacrylat-Einheiten. Derartige Basen werden im Handel beispielsweise unter der Markenkennzeichnung Eudragit® E100 oder Eudragit® E PO angeboten.

Das in der erfindungsgemäßen Zusammensetzung enthaltene Phospholipid ist natürlichen Ursprungs oder synthetisch hergestellt. Aus natürlichen Quellen stammendes Phospholipid besteht in der Regel selbst nach der Aufreinigung aus einem Gemisch von voneinander verschiedenen Phospholipiden, die mit unterschiedlichen Gewichtsanteilen in dem Gemisch enthalten sind. Das Phospholipid liegt in der erfindungsgemäßen Zusammensetzung vorzugsweise in nicht-hydrierter Form vor. Alternativ dazu ist es bevorzugt, dass das Phospholipid in der erfindungsgemäßen Zusammensetzung in hydrierter Form enthalten ist.

Erfindungsgemäß bevorzugtes Phopholipid ist aus Ei oder Soja gewonnenes Phospholipid.

Das in der erfindungsgemäßen Zusammensetzung enthaltene Phospholipid kann jedes Phospholipid oder Gemisch von Phospholipiden sein, das dem Fachmann für den erfindungsgemäßen Zweck als geeignet bekannt ist.

Erfindungsgemäß bevorzugtes Phospholipid ist ausgewählt aus der Gruppe bestehend aus Phosphatidylcholin, Phosphatidylethanolamin, Phosphatidylserin, Phosphatidylinositol, Phosphatidylglycerol und Lysophospholipid. Darüber hinaus ist es erfindungsgemäß bevorzugt, dass das Phospholipid ein Gemisch von zwei oder mehr der in der vorstehenden Gruppe genannten Phospholipide ist.

Entsprechend einer besonders bevorzugten Ausführungsform der erfindungsgemäßen Zusammensetzung ist es vorgesehen, dass das Phospholipid ein Phosphatidylcholin ist oder ein Phosphatidylcholin umfasst.

Gemäß einer weiter bevorzugten Ausführungsform der erfindungsgemäßen Zusammensetzung ist es vorgesehen, dass das Verhältnis der Masse der Zusammensetzung an Phospholipid zur Masse der Zusammensetzung an Wirkstoff 0,001 bis 100 beträgt, bevorzugt 0,1 bis 20 und besonders bevorzugt 1 bis 10. Bei einem Phospholipid/Wirkstoff-Massenverhältnis der Zusammensetzung unterhalb von 0,001 kann keine signifikante Verbesserung der Löslichkeit des Wirkstoffs beobachtet werden, oberhalb eines Massenverhältnisses von 100 hingegen keine weitere Verbesserung der Löslichkeit.

Entsprechend einer weiter bevorzugten Ausführungsform der erfindungsgemäßen Zusammensetzung ist es vorgesehen, dass das Verhältnis der Masse der Zusammensetzung an Phospholipid zur Masse der Zusammensetzung an Maltodextrin 0,01 bis 50 beträgt, vorzugsweise 0,1 bis 10. Dadurch kann eine verfahrenstechnisch einfache und damit kostengünstige Verarbeitung der Zusammensetzung zu Darreichungsformen mit homogener Wirkstoffverteilung gewährleistet werden.

Gemäß einer weiter bevorzugten Ausführungsform der erfindungsgemäßen Zusammensetzung ist es vorgesehen, dass die Zusammensetzung frei von einem Tensid ist, welches nicht der Klasse der Phospholipide zuzuordnen ist.

Es wurde festgestellt, dass in der erfindungsgemäßen Zusammensetzung eine unerwünschte Aggregation des hydrophoben Wirkstoffs auch in dem Fall, in welchem der Wirkstoff in molekular-, nano- und/oder mikrodisperser Form in der Zusammensetzung vorliegt, kaum stattfindet. Auf den Einsatz weiterer Aggregationsverhinderer wie beispielsweise herkömmliche Tenside kann somit verzichtet werden. Unter herkömmlichen Tensiden werden dabei vorzugsweise nichtionische Tenside, kationische Tenside, amphotere Tenside und anionische Tenside verstanden, die frei von einer Phosphatgruppe sind.

Entsprechend einer weiter bevorzugten Ausführungsform der erfindungsgemäßen Zusammensetzung ist es vorgesehen, dass die Zusammensetzung eine orale Zusammensetzung ist.

Um die Stabilität der erfindungsgemäßen Zusammensetzung weiter zu erhöhen, ist es gemäß einer weiter bevorzugten Ausführungsform der Erfindung vorgesehen, dass die Zusammensetzung ein Antioxidationsmittel umfasst. Erfindungsgemäß besonders bevorzugte Antioxidationsmittel sind Ascorbylpalmitat und Vitamin E, wobei Ascorbylpalmitat am meisten bevorzugt ist. Der Anteil der erfindungsgemäßen Zusammensetzung an Antioxidationsmittel liegt vorzugsweise in einem Bereich von 0,01 bis 5 Gew.-%.

Entsprechend einer weiter bevorzugten Ausführungsform der erfindungsgemäßen Zusammensetzung ist es vorgesehen, dass das Phospholipid und das Maltodextrin in der Zusammensetzung in einer Form vorliegen, in welcher das Phospholipid von dem Maltodextrin geträgert ist. Die Trägerung des Phospholipids durch Maltodextrin wirkt einer Zersetzung des Phospholipids besonders wirksam entgegen. Darüber hinaus kann das geträgertes Phospholipid einfacher verarbeitet werden als freies Phospholipid.

Von Maltodextrin geträgerte Phospholipide können beispielsweise hergestellt werden, indem ein Gemisch von Wasser, Phospholipid und Maltodextrin hergestellt und dieses Gemisch getrocknet wird, beispielsweise durch Sprühtrocknung.

Gemäß einer weiter bevorzugten Ausführungsform der erfindungsgemäßen Zusammensetzung ist es vorgesehen, dass in der Zusammensetzung das Phospholipid in Form einer festen Lösung in dem Maltodextrin enthalten ist. Eine derartige feste Lösung kann beispielsweise hergestellt werden, indem das Phospholipid und das Maltodextrin gemeinsam in einem geeigneten Lösungsmittel gelöst werden und die Lösung anschließend getrocknet wird, beispielsweise mittels Sprühtrocknung. Unter dem Ausdruck "feste Lösung" ist in diesem Zusammenhang die weiter unten in Bezug auf Wirkstoffe angegebene Definition für "feste Lösung" analog zu verstehen.

Entsprechend einer weiter bevorzugten Ausführungsform der erfindungsgemäßen Zusammensetzung ist es vorgesehen, dass der Wirkstoff in Form einer festen Lösung in der Zusammensetzung enthalten ist. Der Begriff "feste Lösung" bezeichnet hier einen Zustand, in dem der Wirkstoff mikrodispers, nanodispers oder im Idealfall molekulardispers in einer festen Matrix verteilt enthalten ist. Eine feste Lösung eines schwerlöslichen Wirkstoffs führt zu einer verbesserten Freisetzung des Wirkstoffs. Eine wichtige Anforderung an solche feste Lösungen ist, dass sie auch bei Lagerung über längere Zeit stabil sind, d.h., dass der Wirkstoff nicht auskristallisiert. Weiterhin ist auch die Kapazität der festen Lösung, mit anderen Worten die Fähigkeit der Ausbildung von stabilen festen Lösungen mit möglichst hohen Wirkstoffgehalten, von Bedeutung.

Gemäß einer weiter bevorzugten Ausführungsform der erfindungsgemäßen Zusammensetzung ist es vorgesehen, dass der Wirkstoff in dem Phospholipid und/oder in dem Maltodextrin gelöst enthalten ist. Gemäß dieser bevorzugten Ausführungsform der Erfindung ist der hydrophobe Wirkstoff in einer Matrix gelöst enthalten, wobei die Matrix von dem Maltodextrin und/oder von dem Phospholipid gebildet ist. Eine derartige feste Lösung kann beispielsweise hergestellt werden, indem das Phospholipid, das Maltodextrin und der hydrophobe Wirkstoff gemeinsam in einem geeigneten Lösungsmittel gelöst werden und die Lösung anschließend getrocknet wird, beispielsweise durch Sprühtrocknung oder Gefriertrocknung.

Entsprechend einer weiter bevorzugten Ausführungsform der erfindungsgemäßen Zusammensetzung ist es vorgesehen, dass der Wirkstoff in der Zusammensetzung in mikronisierter Form enthalten ist.

Zur Darreichung der erfindungsgemäßen Zusammensetzung ist es gemäß einer weiter bevorzugten Ausführungsform der Erfindung vorgesehen, dass die Zusammensetzung eine Tablette, eine Granule, ein Pellet, eine Kapsel oder ein Pulver ist.

Der Gehalt einer Darreichungform auf der Basis der erfindungsgemäßen Zusammensetzung an Wirkstoff beträgt vorzugsweise von 0,01 mg bis 1500 mg, bevorzugt von 10 mg bis 100 mg und mehr bevorzugt von 20 mg bis 80 mg.

Entsprechend einer weiter bevorzugten Ausführungsform der erfindungsgemäßen Zusammensetzung ist es vorgesehen, dass die Zusammensetzung zumindest einen Hilfsstoff umfasst. Erfindungsgemäß bevorzugte Hilfsstoffe sind Füllstoffe, insbesondere Lactosemonohydrat, Bindemittel, insbesondere mikrokristalline Cellulose, Sprengmittel, insbesondere Stärke oder Crosscarmellose-Na, und Schmiermittel, insbesondere Mg-Stearat.

Die vorliegende Erfindung betrifft ferner ein Verfahren zur Herstellung einer erfindungsgemäßen pharmazeutischen Zusammensetzung, umfassend die Schritte:
- Vermischen eines hydrophoben Wirkstoffs mit einem Phospholipid und einem Maltodextrin, wobei ein Gemisch erhalten wird;
- Versetzen des Gemisches mit einer Flüssigkeit;
- Granulieren des mit der Flüssigkeit versetzten Gemisches.

Nach dem Granulieren können die Granülen mit jeder dem Fachmann aus dem Stand der Technik als für den erfindungsgemäßen Zweck geeigneten Methode getrocknet werden.

Die vorliegende Erfindung betrifft ein weiteres Verfahren zur Herstellung einer erfindungsgemäßen pharmazeutischen Zusammensetzung, umfassend die Schritte:
- Vermischen eines hydrophoben Wirkstoffs mit einem Phospholipid und einem Maltodextrin, wobei ein Gemisch erhalten wird;
- Kompaktieren des Gemisches.

Das Gemisch kann zu Pellets, Tabletten oder größeren Einheiten kompaktiert werden, wobei letztere für die Herstellung von Darreichungsformen zerkleinert werden müssen.

Die vorliegende Erfindung betrifft noch ein weiteres Verfahren zur Herstellung einer erfindungsgemäßen pharmazeutischen Zusammensetzung, umfassend den Schritt:
- Vermischen eines hydrophoben Wirkstoffs mit einem Phospholipid und einem Maltodextrin.

Das so erhaltene Gemisch kann zur Herstellung von Darreichungsformen beispielsweise in Kapseln abgefüllt werden.

Die vorliegende Erfindung betrifft noch ein weiteres Verfahren zur Herstellung einer erfindungsgemäßen pharmazeutischen Zusammensetzung, umfassend die Schritte:
- Herstellen eines Gemisches, umfassend ein Lösungsmittel, einen hydrophoben Wirkstoff, ein Phospholipid und ein Maltodextrin, wobei zumindest der Wirkstoff und das Phospholipid in dem Lösungsmittel gelöst enthalten sind;
- Entfernen des Lösungsmittels aus dem Gemisch.

Mittels dieses erfindungsgemäßen Verfahrens können feste Zusammensetzungen erhalten werden, in welcher der Wirkstoff gelöst enthalten ist.

Die vorliegende Erfindung betrifft ferner die Verwendung einer erfindungsgemäßen Zusammensetzung als Arzneimittel.

Die vorliegende Erfindung betrifft ferner die Verwendung einer erfindungsgemäßen Zusammensetzung enthaltend Ziprasidon zur Herstellung eines Medikamentes zur Behandlung von neuroleptischen Erkrankungen.

Die vorliegende Erfindung betrifft ferner die Verwendung einer erfindungsgemäßen Zusammensetzung enthaltend Telmisartan zur Herstellung eines Medikamentes zur Behandlung von Bluthochdruck.

Die vorliegende Erfindung betrifft ferner die Verwendung eines Pulvers, enthaltend Partikel, die ein Phospholipid und ein Maltodextrin umfassen, zur Herstellung einer erfindungsgemäßen pharmazeutischen Zusammensetzung.

Die nachstehende Beschreibung bevorzugter Ausführungsbeispiele der erfindungsgemäßen pharmazeutischen Zusammensetzung sowie bevorzugter Ausführungsformen erfindungsgemäßer Verfahren zur Herstellung einer erfindungsgemäßen Zusammensetzung dient der Erläuterung der Erfindung.

### Beispiele 1 bis 4:

In der Tabelle 1 sind Zusammensetzungen von Kapseln als Ausführungsbeispiele für eine erfindungsgemäße pharmazeutische Zusammensetzung angegeben.

**Tabelle 1:**

| Beispiel-Nr. | 1 | 2 | 3 | 4 |
|---|---|---|---|---|
| | [mg] | [mg] | [mg] | [mg] |
| Ziprasidon-hydrochlorid (monohydrat) | 20,00 | 40,00 | 60,00 | 80,00 |
| Cellactose | 67,73 | 135,46 | 203,19 | 270,92 |
| Hypromellose | 1,50 | 3,00 | 4,50 | 6,00 |
| Wasser¹⁾ (demineralisiert) | q.s. | q.s. | q.s. | q.s. |
| Phospholipid | 2,00 | 4,00 | 6,00 | 8,00 |
| Maltodextrin | 6,00 | 12,00 | 18,00 | 24,00 |
| Magnesiumstearat | 2,25 | 4,50 | 6,75 | 9,00 |
| Gewicht des Kapselinhalts | 99,48 | 198,96 | 298,44 | 397,92 |

| | | | | |
|---|---|---|---|---|
| ¹⁾ nicht im Endprodukt enthalten | | | | |

Die Kapseln gemäß der Ausführungsbeispiele 1 bis 4 wurden hergestellt, indem zunächst Cellactose (zusammengesetzt aus 25 Gew.-% Pulvercellulose und 75 Gew.-% Lactose-Monohydrat) mit einer wässrigen Lösung enthaltend Hypromellose granuliert wurde. Das getrocknete Granulat wurde dann mit dem Wirkstoff, einem pulverförmigen Phospholipid/Maltodextrin-Gemisch und Magnesiumstearat vermischt und die Mischung in Kapseln eingefüllt.

Das pulverförmige Phospholipid/Maltodextrin-Gemisch wurde hergestellt, indem ein Gemisch von Phospholipid (Zusammensetzung: Phosphatidylcholin ca. 88 Gew.-%, Lysophosphatidylcholin ca. 12 Gew.-%; gewonnen aus Soja-Lecithin durch Extraktion mit Ethanol und Aufreinigen des Extrakts mittels Chromatographie), und Maltodextrin in Wasser angerührt und anschließend sprühgetrocknet wurde.

### Beispiele 5 bis 8:

In der Tabelle 2 sind weitere Zusammensetzungen von Kapseln als Ausführungsbeispiele für eine erfindungsgemäße pharmazeutische Zusammensetzung angegeben.

**Tabelle 2:**

| Beispiel-Nr. | 5 | 6 | 7 | 8 |
|---|---|---|---|---|
| | [mg] | [mg] | [mg] | [mg] |
| Ziprasidon hydrochlorid (monohydrat) | 20,00 | 40,00 | 60,00 | 80,00 |
| Lactose-Monohydrat | 69,23 | 138,46 | 207,69 | 276,92 |
| Stearinsäure | 3,00 | 6,00 | 9,00 | 12,00 |
| Siliziumoxid | 7,27 | 14,54 | 21,81 | 29,08 |
| Phospholipid | 0,50 | 1,00 | 1,50 | 2,00 |
| Maltodextrin | 1,50 | 3,00 | 4,50 | 6,00 |
| Gewicht des Kapselinhalts | 101,50 | 203,00 | 304,50 | 406,00 |

Die Kapseln gemäß der Ausführungsbeispiele 5 bis 8 wurden hergestellt, indem Lactose-Monohydrat mit dem Wirkstoff, einem pulverförmigen Phospholipid/Maltodextrin-Gemisch (Herstellung siehe Beispiele 1 bis 4), Siliziumoxid und Stearinsäure vermischt wurde, das resultierende Gemisch zu Pellets kompaktiert und die Pellets in Kapseln abgefüllt wurden.

### Beispiele 9 bis 12:

In der Tabelle 3 sind weitere Zusammensetzungen von Kapseln als Ausführungsbeispiele für eine erfindungsgemäße pharmazeutische Zusammensetzung angegeben.

**Tabelle 3:**

| Beispiel-Nr. | 9 | 10 | 11 | 12 |
|---|---|---|---|---|
| | [mg] | [mg] | [mg] | [mg] |
| Ziprasidonhydrochlorid (monohydrat) | 20,00 | 40,00 | 60,00 | 80,00 |
| Phospholipid | 40,00 | 80,00 | 120,00 | 160 |
| Maltodextrin | 120,00 | 240,00 | 360,00 | 480,00 |
| Lösungsmittel¹⁾ | q.s. | q.s. | q.s. | q.s. |
| Lactose | 20,00 | 40,00 | 60,00 | 80,00 |
| Mannitol | 18,00 | 36,00 | 54,00 | 72,00 |
| Siliziumoxid | 2,00 | 4,00 | 6,00 | 8,00 |
| Gewicht des Kapselinhalts | 220,00 | 440,00 | 660,00 | 880,00 |

| | | | | |
|---|---|---|---|---|
| ¹⁾ nicht im Endprodukt enthalten | | | | |

Die Kapseln gemäß der Ausführungsbeispiele 9 bis 12 wurden hergestellt, indem zunächst ein Gemisch aus dem Wirkstoff, einem Phospholipid (Zusammensetzung: Phosphatidylcholine ca. 88 Gew.-%, Lysophosphatidylcholine ca. 12 Gew.-%; gewonnen aus Soja-Lecithin durch Extraktion mit Ethanol und Aufreinigen des Extrakts mittels Chromatographie), einem Maltodextrin und Dichlormethan angesetzt und das resultierende Gemisch sprühgetrocknet wurde. Das durch Sprühtrocknung erhaltene Material wurde mit den Komponenten Lactose, Mannitol und Siliziumoxid vermischt und das resultierende Gemisch in Kapseln gefüllt.

### Beispiele 13 bis 16:

In der Tabelle 4 sind weitere Zusammensetzungen von Kapseln als Ausführungsbeispiele für eine erfindungsgemäße pharmazeutische Zusammensetzung angegeben.

**Tabelle 4:**

| Beispiel-Nr. | 13 | 14 | 15 | 16 |
|---|---|---|---|---|
| | [mg] | [mg] | [mg] | [mg] |
| Ziprasidon-hydrochlorid (monohydrat) | 20,00 | 40,00 | 60,00 | 80,00 |
| Phospholipid | 40,00 | 80,00 | 120,00 | 160 |
| Maltodextrin | 120,00 | 240,00 | 360,00 | 480,00 |
| Lactose | 20,00 | 40,00 | 60,00 | 80,00 |
| Lösungsmittel ¹⁾ | q.s. | q.s. | q.s. | q.s. |
| Mannitol | 18,00 | 36,00 | 54,00 | 72,00 |
| Siliziumoxid | 2,00 | 4,00 | 6,00 | 8,00 |
| Gewicht des Kapselinhalts | 220,00 | 440,00 | 660,00 | 880,00 |

| | | | | |
|---|---|---|---|---|
| ¹⁾ nicht im Endprodukt enthalten | | | | |

Die Kapseln gemäß der Ausführungsbeispiele 13 bis 16 wurden hergestellt, indem zunächst ein Gemisch aus dem Wirkstoff, einem Phospholipid (Zusammensetzung: siehe Beispiele 9 bis 12), einem Maltodextrin und Dichlormethan angesetzt wurde. Dieses Gemisch wurde auf ein Wirbelbett von Lactose aufgesprüht. Die so erhaltenen Granülen wurden mit Mannitol und Siliziumoxid vermischt und das resultierende Gemisch in Kapseln abgefüllt.

## Patentansprüche

1. Feste pharmazeutische Zusammensetzung, umfassend einen hydrophoben Wirkstoff, ein Phospholipid sowie ein Maltodextrin.

2. Zusammensetzung nach Anspruch 1, **dadurch gekennzeichnet, dass** der Wirkstoff Zlprasidon ist oder ein pharmazeutisch annehmbares Salz davon.

3. Zusammensetzung nach Anspruch 1 oder 2, **dadurch gekennzeichnet, dass** die Zusammensetzung eine Säure umfasst.

4. Zusammensetzung nach Anspruch 1, **dadurch gekennzeichnet, dass** der Wirkstoff Telmisartan ist oder ein pharmazeutisch annehmbares Salz davon.

5. Zusammensetzung nach Anspruch 1 oder 4, **dadurch gekennzeichnet, dass** die Zusammensetzung eine Base umfasst.

6. Zusammensetzung nach einem der voranstehenden Ansprüche, **dadurch gekennzeichnet, dass** das Phospholipid ein Phosphatidylcholin ist.

7. Zusammensetzung nach einem der voranstehenden Ansprüche, **dadurch gekennzeichnet, dass** das Verhältnis der Masse der Zusammensetzung an Phospholipid zur Masse der Zusammensetzung an Wirkstoff von 0,001 bis 100 beträgt.

8. Zusammensetzung nach einem der voranstehenden Ansprüche, **dadurch gekennzeichnet, dass** das Verhältnis der Masse der Zusammensetzung an Phospholipid zur Masse der Zusammensetzung an Maltodextrin von 0,01 bis 50 beträgt.

9. Zusammensetzung nach einem der voranstehenden Ansprüche, **dadurch gekennzeichnet, dass** das Phospholipid und das Maltodextrin in der Zusammensetzung in einer Form vorliegen, in welcher das Phospholipid von dem Maltodextrin geträgert ist.

10. Zusammensetzung nach einem der voranstehenden Ansprüche, **dadurch gekennzeichnet, dass** in der Zusammensetzung das Phospholipid in Form einer festen Lösung in dem Maltodextrin enthalten ist.

11. Zusammensetzung nach einem der voranstehenden Ansprüche, **dadurch gekennzeichnet, dass** der Wirkstoff in Form einer festen Lösung in der Zusammensetzung enthalten ist.

12. Zusammensetzung nach Anspruch 11, **dadurch gekennzeichnet, dass** der Wirkstoff in dem Phospholipid und/oder in dem Maltodextrin gelöst enthalten ist.

13. Zusammensetzung nach einem der Ansprüche 1 bis 10, **dadurch gekennzeichnet, dass** der Wirkstoff in der Zusammensetzung in mikronisierter Form enthalten ist.

14. Verfahren zur Herstellung einer pharmazeutischen Zusammensetzung nach einem der voranstehenden Ansprüche, umfassend die Schritte
- Vermischen eines hydrophoben Wirkstoffs mit einem Phospholipid und einem Maltodextrin, wobei ein Gemisch erhalten wird;
- Versetzen des Gemisches mit einer Flüssigkeit;
- Granulieren des mit der Flüssigkeit versetzten Gemisches;
oder umfassend die Schritte
- Vermischen eines hydrophoben Wirkstoffs mit einem Phospholipid und einem Maltodextrin, wobei ein Gemisch erhalten wird;
- Kompaktieren des Gemisches;
oder umfassend den Schritt
- Vermischen eines hydrophoben Wirkstoffs mit einem Phospholipid und einem Maltodextrin;
oder umfassend die Schritte
- Herstellen eines Gemisches, umfassend ein Lösungsmittel, einen hydrophoben Wirkstoff, ein Phospholipid und ein Maltodextrin, wobei zumindest der Wirkstoff und das Phospholipid in dem Lösungsmittel gelöst enthalten sind;
- Entfernen des Lösungsmittels aus dem Gemisch.

15. Verwendung eines Pulvers, enthaltend Partikel, die ein Phospholipid und ein Maltodextrin umfassen, zur Herstellung einer pharmazeutischen Zusammensetzung nach einem der Ansprüche 1 bis 13.
